# DEMANDE DE BREVET EUROPEEN

(11) **EP 0 739 635 A1**
(43) Date de publication de la demande: **30.10.1996**
(21) Numéro de dépôt: 96400798.3
(22) Date de dépôt: 12.04.1996
(51) Int. Cl.: A61L 15/18, A61L 15/46

(54) **Composition absorbante destinée à la réalisation d'article d'hygiène du type linges, couches, changes ne développant pas d'odeurs incommodantes**

(30) Priorité: 18.04.1995 FR 9504584
(71) Demandeur: ELF ATOCHEM S.A., 92800 Puteaux (FR)
(72) Inventeur: Gancet, Christian, 64140 Lons (FR); Seris, Jean-Louis, 64110 Jurancon (FR); Cuny, Nathalie, 78420 Carrieres/Seine (FR); Lescure, Monique, 64000 Pau (FR)
(74) Mandataire: Haicour, Philippe

(57) **Abrégé**

Ces compositions sont constituées de polymères superabsorbants du type polyacrylique et de certains dérivés boriques, en particulier le tétraborate de sodium. Les articles sanitaires incorporant ces compositions ne développent pas d'odeurs incommodantes, ammoniacales ou autres.

## Description

L'invention a trait à la réalisation d'articles sanitaires destinés à absorber et retenir les liquides corporels.

Lorsque l'article absorbant en place est imprégné de tels liquides corporels, en particulier d'urine, il s'y développe des odeurs puissantes et incommodantes. Parmi celles-ci dominent des odeurs ammoniacales dues à l'ammoniac provenant de l'hydrolyse de l'urée par les uréases des bactéries *(Proteus, Acinetobacter,* etc.) présentes sur la peau et dans le tube digestif.

Dans le but de supprimer ces odeurs, on a fait largement appel à des adsorbants d'odeurs ou d'ammoniac (US 3,340,875, Scott Paper Company) associés ou non à des déodorants, des parfums, etc. On a aussi préconisé l'utilisation de tampons de pH (WO-A-94 25077), d'oxydants (eau oxygénée, bioxyde de chlore), de biocides (métaux ou de cations métalliques), ainsi que de bactéricides (ammoniums quaternaires en particulier), d'antibiotiques, de complexants, de tensioactifs, tant seuls qu'associés entre eux. Ces divers produits posent le problème général de leur action irritante sur la peau et les muqueuses. Les absorbants d'odeurs ou d'ammoniac sont certainement moins dangereux à cet égard, mais ils laissent libre champ à une prolifération bactérienne qui reste préoccupante et qu'il conviendrait de contrôler dès l'origine. La voie qui semblait s'imposer pour le contrôle de l'émission bactérienne d'ammoniac à partir de l'urée, consiste à inhiber les enzymes qui en sont responsables, c'est-à-dire les uréases.

L'utilisation des inhibiteurs d'uréase à activité faible ou modérée n'a été jusqu'à présent envisagée qu'en association avec des capteurs d'ammoniac (JP 61-179155). Certains inhibiteurs d'uréases puissants, comme les dérivés d'acide hydroxamique (US 3,920,015, Allied Chem. Corp.) sont intéressants, mais leur toxicité les écarte de l'emploi visé: D'autres, comme le phénylphosphorodiamidate (PPDA) ou le diméthyldithiocarbamante (DTC), sont des composés très efficaces contre la formation d'ammoniac, mais donnent naissance lors de leur utilisation, à d'autres odeurs soufrées ou alliacées très désagréables, qui sont peut-être liées, en partie au moins, à leur propre décomposition par les flores bactériennes présentes.

La recherche d'une solution à ce problème est d'autant plus pressante que, de nos jours, on augmente très sensiblement la capacité d'absorption pour les liquides corporels des articles de protection en leur incorporant des polymères superabsorbants (SAP), en particulier les polymères et copolymères hydrophiles d'acide acrylique, et que du même coup, on augmente la durée de leur maintien en place, toutes conditions favorisant le développement de l'activité microbienne et enzymatique et des odeurs qui en résultent.

On vient maintenant de trouver qu'il est possible de formuler des polymères superabsorbants avec certains dérivés boriques pour en faire des compositions qui bien qu'imbibées d'urine ou de liquides biologiques en contenant et maintenues dans les conditions d'utilisation pourtant propices à un développement bactérien, ne donnent lieu ni à dégagement important d'ammoniac, ni à émissions d'odeurs repoussantes ou simplement désagréables, et qui communiquent cette propriété aux articles d'hygiène qui en contiennent.

L'invention consiste ainsi en une composition superabsorbante destinée à la réalisation d'article d'hygiène du type linges, couches, changes ne développant pas d'odeurs incommodantes, comprenant un polymère superabsorbant pour l'eau, les solutions salines et les liquides corporels et du tétraborate de sodium ou du métaborate de sodium dans la proportion de 0,1 à 10% de dérivé borique, de préférence 0,5 à 5%, par rapport à la composition superabsorbante.

Les polymères superabsorbants au sens de la présente invention sont des polymères qui résultent de la polymérisation avec réticulation partielle de monomères éthyléniquement insaturés hydrosolubles, en particulier les acides acryliques et méthacryliques et leurs sels alcalins, qu'ils soient obtenus par un procédé de polymérisation en solution ou en suspension inverse. Ces polymères sont doués d'une très grande capacité d'absorption et de rétention de l'eau et des solutions aqueuses, et aujourd'hui largement répandus dans le commerce sous forme de poudres avec des granulométries restant comprises entre 100 et 800µm. La littérature en est très riche ; on pourra consulter par exemple EP-A-0312952 (The Dow Chem. Co.) et EP-A-0441507 (Sumitomo Seika Chem.).

Au sens de la présente invention, le tétraborate de sodium (Na₂B₄O₇) est le sel anhydre, pentahydraté ou décahydraté (borax) et le métaborate (NaBO₂) est considéré également indépendamment de son degré d'hydratation. Ces composés boriques sont mélangés au polymère en poudre dans des proportions indiquées plus haut.

La préparation de la composition selon l'invention est très aisée, puisqu'il suffit d'incorporer par simple mélange à la poudre de polymère superabsorbant le dérivé borique en poudre, de préférence avec une granulométrie également comprise entre 100 et 800 µm. On a toutefois constaté que si le dérivé borique était introduit au sein des particules de polymère superabsorbant, on développait, à concentration équivalente, une efficacité anti-odeur plus grande que par simple mélange des poudres. D'où une variante du procédé, qui fait également partie de l'invention, qui consiste à gonfler la poudre de polymère à l'aide d'une solution aqueuse de dérivé borique, puis à évaporer l'eau ainsi introduite.

Les compositions superabsorbantes de l'invention se gélifient au contact de l'eau, des solutions aqueuses salines ou des liquides corporels comme les superabsorbants de l'art antérieur, et les gels ainsi formés se comportent de façon sensiblement identique. Elles ne souffrent d'aucune contre-indication, les dérivés boriques qui sont les moyens de l'invention étant à juste titre considérés comme inoffensifs, et largement utilisés dans diverses compositions antiseptiques douces, à des teneurs de l'ordre de 1 % en poids. On les utilise en lieu et place des superabsorbants ordinaires dans la fabrication des articles d'hygiène comme les changes complets ou des couches-culottes pour bébés, pour jeunes enfants, pour adultes ou pour vieillards des deux sexes. Cette utilisation constitue également un objet de la présente invention.

L'appréciation de l'efficacité réelle de produits anti-odeur est chose délicate. Puisque l'invention s'inscrit dans un contexte d'inhibiteurs d'uréase, on opère donc un classement général des composés envisageables pour leur activité anti-uréase candidats par un indice IC50 selon les résultats d'un test enzymatique qu'on trouvera décrit plus loin. Puisque le processus générateur d'odeurs est un processus biologique, on doit pouvoir estimer l'efficacité de divers inhibiteurs d'uréase pour leur aptitude à limiter l'émission biologique par micro-organismes fermentant l'urée d'au moins le composé odorant le plus abondant, en la circonstance l'ammoniac, cette efficacité s'entendant de l'inhibiteur tel quel ou en présence des diverses substances auxquelles il est associé dans la réalisation des couches et autres articles sanitaires, en particulier les polymères superabsorbants. Les résultats de ce test est assez bien corrélés avec les valeurs des IC50. Il faut enfin et surtout pouvoir décider du résultat global satisfaisant par des tests olfactifs dans des conditions qui simulent acceptablement les conditions d'utilisation des produits dans lesquels la composition superabsorbante présumée inhibitrice d'odeurs est incorporée. On les réalise en imbibant d'urine un change dans des conditions standardisées, en étuvant l'ensemble à douce température et en soumettant l'objet à un panel de nez pour l'appréciation globale de ses éventuelles mauvaises odeurs.

De tels tests sont décrits dans les exemples données ci-dessous, lesquels illustrent l'efficacité inattendue des produits selon l'invention. Dans ces essais, le polymère superabsorbant utilisé est un acide polyacrylique partiellement neutralisé commercialisé sous le nom d'AQUA-KEEP®D (Elf Atochem S.A.).

### Exemple 1 : IC50 de quelques molécules réputées fonctionner comme inhibiteurs d'uréase.

Le test consiste à décomposer une solution neutre d'urée à 1 g/l (0,016 M) dans un tampon triéthanolamine 0,1 M, additionnée ou non du composé dont on veut estimer l'activité anti-uréase, par une solution d'uréase de soja (U1500 de SIGMA) à 1 mg/ml dans le tampon + 10 mM en glutathion dans les conditions suivantes. On mélange 400 µl de solution d'uréase et 10 ml de solution d'urée. Le mélange est maintenu à 37°C. Des prélèvements de 1 ml sont opérés au cours du temps et titrés en NH3 par la méthode de Nessler: chaque échantillon de 1 ml est ajouté à 24,5 ml de réactif de Nessler dilué (0,5 pour 24) et après 10 minutes d'incubation, on réalise une mesure d'absorbance à 425 nm. On détermine un indice IC50 qui est la concentration d'inhibiteur dans le mélange, exprimée en µM, capable de réduire de 50 % l'activité uréase dans les conditions du test.

Le tableau suivant rapporte les valeurs IC50 pour quelques inhibiteurs d'uréase connus de la littérature.

| Composé | IC50 (µM) |
|---|---|
| Phénylphosphorodiamidate (PPDA) | 0,05 |
| Acide borique | 450 |
| Métaborate de sodium | 260 |
| Tétraborate de sodium 5H₂O | 160 |
| Pyrophosphate de sodium | 8.000 |
| Hydroquinone | 500 |
| Diméthyldithiocarbamate (DTC) | 10 |
| Acide salicylhydroxamique | 310 |
| Acide glycolique | 450 |
| Acide acétohydroxamique | 600 |
| Acide nitrilotriacétique | 5.000 |

### Exemple 2 : Synergie SAP / pentahydrate de tétraborate de sodium

On procède à la détermination de l'indice IC50 comme décrit plus haut. Les essais sont menés par comparaison à la fois sur un milieu témoin, et sur des milieu contenant respectivement uniquement du SAP, du pentahydrate de tétraborate de sodium (Na₂B₄O₇ 5H₂O) et du SAP additivé de 3,9% en poids de pentahydrate de tétraborate de sodium, que l'on a obtenu en mélangeant 29 Kg de Na₂B₄O₇ 5H₂O à 718,5 Kg de polymère superabsorbant de façon à obtenir finalement 750 Kg de composition boratée. Les résultats sont consignés dans le tableau qui suit.

| Composition | Activité uréase résiduelle % |
|---|---|
| Témoin (sans SAP et sans inhibiteur) | 100 |
| SAP seul (10 mg/15 ml de milieu) | 100 |
| SAP seul (100 mg/15 ml de milieu) | 100 |
| SAP boraté (10 mg/15 ml de milieu) | 50 |
| SAP boraté (100 mg/15 ml de milieu) | 0 |

L'efficacité du SAP boraté est démontrée de façon évidente par le test.

On a noté que la courbe de l'activité uréase en fonction de la teneur en SAP additivé dans le milieu était une courbe régulière. Dans ces conditions, le point d'activité uréase résiduelle 50% pour ce SAP à 3,9% de tétraborate pentahydrate peut s'interpréter en terme d'lC50 rapporté au tétraborate, et se calcule à 90 µM. On comparera ce chiffre à la valeur de 160 µM pour le tétraborate pentahydrate seul du tableau de l'exemple 1 pour constater que l'IC50 attribuable au tétraborate de sodium pentahydrate en présence de SAP est supérieur à celui que l'on calcule avec le tétraborate seul. Cet effet de synergie entre le SAP et le tétraborate quant à son activité anti-uréase est très inattendu et reste inexpliqué.

### Exemple 3 : Effet inhibiteur de formation d'ammoniac.

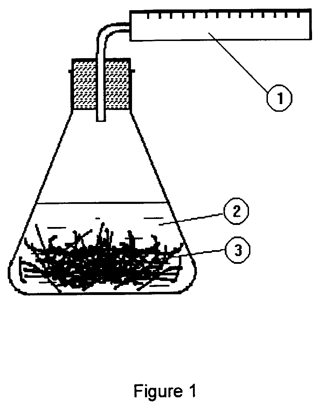

Dans un vase d'Erlenmeyer de 100 ml équipé (voir fig. 1) d'un tube dosimétrique Prolabo n° 02.436.112 ①(ce sont des tubes gradués en verre contenant un réactif de l'ammoniac qui vire du violet au jaune, la position de la démarcation entre les deux colorations donnant une indication sur la quantité d'ammoniac dégagée), on introduit de l'urine ② et un mélange de pulpe de cellulose défibrée et de SAP ③ dans les proportions suivantes:

| | |
|---|---|
| urine fraîche | 50 ml |
| urine fermentée (inoculum) | 2 ml |
| superabsorbant | 0,5 g |
| pulpe de cellulose défibrée (fluff) | 0,5 g |

l'inoculum est nécessaire, parce que l'urine fraîche est stérile. On prépare cet inoculum de la façon suivante: à 100 ml d'urine fraîche, on ajoute 0,25 g d'urée et 1 g de fluff souillé provenant d'un change bébé usagé (on pourrait pour plus de commodité ensemencer le milieu avec, au lieu du fluff usagé, d'une souche de *Bacillus*, *Proteus ou Acinetobacter*). Cette solution, prélevée après 24 heures à température ambiante, constitue l'inoculum.

Dans cet essai, l'inhibiteur d'odeur n'est pas introduit séparément, mais avec le superabsorbant formulé comme indiqué plus haut et avec une teneur dans le superabsorbant telle que sa dose dans le milieu à fermenter soit de 10 fois son IC50.

Le système est abandonné 18 heures à 40°C, après quoi on note la position de la zone de transition de coloration violet/jaune dans le tube dosimétrique, qui fournit ainsi une estimation comparative du dégagement d'ammoniac et donc de l'influence sur ce dégagement des produits soumis à l'essai. Après quoi, on procède avec précaution à l'ouverture de l'Erlenmeyer et l'expérimentateur donne son appréciation sur l'odeur du contenu. Les résultats sont consignés dans le tableau ci-après.

| Support/inhibiteur | Lecture sur le dosimètre | Odeur |
|---|---|---|
| Témoin (ni SAP, ni inhibiteur) | >500 | Ammoniacale forte |
| SAP seul | >500 | Ammoniacale forte |
| SAP + Na₂B₄O₇ 5H₂O | 90 | Pas d'odeur d'ammoniac perceptible. Odeur neutre d'urine fraîche |
| SAP + PPDA | 120 | Odeur désagréable de chou pourri |
| SAP + DTC | 1070 | Odeur désagréable de chou pourri |
| SAP + hydroquinone | 120 | Légère nauséabonde |

### Exemple 4 : essai comparatif d'un traitement anti-odeur par mélange de poudres ou par imprégnation.

Dans le dispositif de l'exemple 3, on introduit une urine synthétique ensemencée par *B. pasteurii* (ATCC 11859), et un mélange de pulpe de cellulose défibrée et de SAP à 4% de borax, l'un étant réalisé par mélange des poudres de SAP et de borax, l'autre par imprégnation de SAP par la quantité nécessaire de solution aqueuse de borax à 80°C (solution à 22°% en poids). On rapporte ci-après les quantités comparées de NH₃ dégagé (en ppm) à différents moments :

| t (heures) | SAP seul | SAP + 4% borax poudre | SAP + 4% borax solution |
|---|---|---|---|
| 0 | 0 | 0 | 0 |
| 6 | 7 | 7 | 7 |
| 8 | 20 | 18 | 10 |
| 10 | hors échelle | 24 | 12 |
| 30 | hors échelle | hors échelle | 13 |

Ces résultats montrent l'avantage, surtout sur de relativement longues durées, de l'imprégnation tétraborique par rapport au simple mélange.

### Exemple 5

L'essai se fait en utilisant comme support la matière de changes ne contenant pas de SAP. On prend un tel change blanc, de format 31 X 11 cm, que l'on ouvre et sur lequel on répartit 4 grammes de SAP sur une surface d'environ 15 X 9 cm. On humecte le centre du change d'un mélange de 50 ml d'urine fraîche et de 2 ml d'inoculum. On enferme le change dans un sac plastique que l'on soude. On place le tout en étude ventilée à 37°C pendant 12 heures. A ce terme, on ouvre le sac et l'on fait estimer l'odeur à un panel de nez (8 personnes). On obtient , dans un système de notation qui confère la note 0 à l'absence totale d'odeur, à 5 pour une très forte odeur d'ammoniac :

| | |
|---|---|
| Change témoin | 4,6 |
| Change avec SAP ordinaire | 4,6 |
| 4% de borax "poudre" | 3,3 |
| 4% de borax "liquide" | 2,8 |

On a ainsi relevé :
une forte odeur d'ammoniaque sur le change témoin,
une forte odeur d'ammoniaque sur le change portant un superabsorbant polyacrylique ordinaire),
aucune odeur d'ammoniaque sur le change avec un superabsorbant polyacrylique contenant 3,9 % de pentahydrate de tétraborate de sodium.
Un essai du même type, mené avec une urine synthétique confirme, avec un plus de netteté, les résultats précédents :

| | |
|---|---|
| Change témoin | 5 |
| Change avec SAP ordinaire | 5 |
| 4% de borax "poudre" | 4 |
| 4% de borax "liquide" | 3 |

## Revendications

1. Composition superabsorbante destiné à la réalisation d'article d'hygiène du type linges, couches, changes ne développant pas d'odeurs incommodantes, caractérisée en ce qu'elle contient:
un polymère superabsorbant résultant de la polymérisation avec réticulation partielle de monomères éthyléniquement insaturés hydrosolubles, en particulier les acides acryliques et méthacryliques et leurs sels alcalins,
du tétraborate de sodium ou du métaborate de sodium, dans la proportion de 0,1 à 10% de dérivé borique, de préférence 0,5 à 5%, par rapport à la composition superabsorbante.

2. Composition selon la revendication 1, caractérisé en ce que le dérivé borique est le tétraborate de sodium sous forme anhydre, pentahydrate ou décahydrate.

3. Composition selon la revendication 1, caractérisé en ce que le dérivé borique est le tétraborate de sodium pentahydrate.

4. Composition selon les revendications 1, 2 ou 3, caractérisée en ce que les granulométries sont comprises entre 100 et 800 µm.

5. Procédé pour la réalisation de compositions superabsorbantes destinées à la réalisation d'articles d'hygiène ne développant pas d'odeurs incommodantes, comportant un polymère superabsorbant résultant de la polymérisation avec réticulation partielle de monomères éthyléniquement insaturés hydrosolubles, en particulier les acides acryliques et méthacryliques et leurs sels alcalins et du tétraborate de sodium ou du métaborate de sodium,, caractérisé en ce qu'on mélange le polymère superabsorbant et le dérivé borique pris chacun sous forme de poudre.

6. Procédé pour la réalisation de compositions superabsorbantes destinées à la réalisation d'articles d'hygiène ne développant pas d'odeurs incommodantes, comportant un polymère superabsorbant résultant de la polymérisation avec réticulation partielle de monomères éthyléniquement insaturés hydrosolubles, en particulier les acides acryliques et méthacryliques et leurs sels alcalins, et du tétraborate de sodium ou du métaborate de sodium, caractérisé en ce qu'on gonfle une poudre de polymère superabsorbant avec une solution aqueuse de dérivé borique, et qu'on élimine l'excès d'eau par évaporation.

7. Utilisation de la composition selon les revendications 1 à 4 dans l'élaboration d'articles d'hygiène comme les changes complets ou des couches-culottes pour bébés, pour jeunes enfants, pour adultes ou pour vieillards des deux sexes.
